# EUROPÄISCHE PATENTSCHRIFT

(11) **EP 0 912 898 B1**
(45) Veröffentlichungstag und Bekanntmachung des Hinweises auf die Patenterteilung: **05.12.2001**
(21) Anmeldenummer: 97939994.6
(22) Anmeldetag: 14.07.1997
(51) Int. Cl.: G01N 33/564, G01N 33/573, A61K 38/45

(54) **IMMUNOLOGISCHES NACHWEISVERFAHREN VON ANTIKÖRPERN, DIE GEGEN GEWEBE-TRANSGLUTAMINASE (tTG) GERICHTET SIND, VERWENDUNG VON tTG ZUR DIAGNOSE UND THERAPIEKONTROLLE SOWIE ORALES PHARMAZEUTISCHES MITTEL ENTHALTEND tTG**
IMMUNOLOGICAL PROCESS FOR DETECTING ANTIBODIES DIRECTED TOWARDS TISSUE TRANSGLUTAMINASE (TTG), USE OF TTG FOR DIAGNOSTIC PURPOSES AND THERAPY CONTROL, AND ORAL PHARMACEUTICAL AGENT CONTAINING TTG
PROCEDE IMMUNOLOGIQUE DE MISE EN EVIDENCE D'ANTICORPS DIRIGES CONTRE LA TRANSGLUTAMINASE TISSULAIRE (TTG), UTILISATION DE LA TTG A DES FINS DE DIAGNOSTIC ET DE CONTROLE DE THERAPIE ET AGENT PHARMACEUTIQUE ORAL CONTENANT DE LA TTG

(30) Priorität: 18.07.1996 DE 19630557
(43) Veröffentlichungstag der Anmeldung: 06.05.1999
(73) Patentinhaber: Schuppan, Detlef, Prof., 12163 Berlin (DE); Dieterich, Walburga, Dr., 91054 Erlangen (DE)
(72) Erfinder: SCHUPPAN, Detlef, D-12163 Berlin (DE); DIETERICH, Walburga, 91054 Erlangen (DE); EHNIS, Tobias, 91054 Erlangen (DE)
(74) Vertreter: Ziebig, Marlene, Dr. Dipl.-Chem.
(86) Internationale Anmeldenummer: EP9703740
(87) Internationale Veröffentlichungsnummer: WO9803872

(56) Entgegenhaltungen:
- C.P.J. MAURY ET AL.: "Autoantibodies to gliadin-binding 90 kDa glycoprotein in coeliac disease." GUT, Bd. 27, Nr. 2, 1986, LONDON UK, Seiten 147-152, XP002052978
- W. DIETERICH ET AL.: "Identification of tissue transglutaminase as the autoantigen of celiac disease." NATURE MEDICINE, Bd. 3, Nr. 7, 1997, NEW YORK NY USA, Seiten 797-801, XP002052979
- U. VOLTA ET AL: 'IgA anti-endomysial antibodies on human umbilical cord tissue for celiac disease screening' DIGESTIVE DISEASES AND SCIENCES Bd. 40, Nr. 9, Seiten 1902 - 1905
- U. VOLTA ET AL: 'IgA anti-endomysial antibody test, a step forward in celiac disease screening' DIGESTIVE DISEASES AND SCIENCES Bd. 36, Nr. 6, Seiten 752 - 756
- A. BÜRGIN-WOLFF ET AL.: 'Antigliadin and antiendomysium antibody determination for coelic disease' ARCHIVES OF DISEASE IN CHILDHOOD Bd. 66, Seiten 941 - 947
- P. ACCETTA ET AL.: 'Anti-Endomysial Antibodies' ARCH DERMATOL Bd. 122, April 1986, Seiten 459 - 461

## Beschreibung

Die Erfindung betrifft ein Verfahren zur Diagnose oder Therapiekontrolle der Sprue oder Zöliakie, in dem Antikörper aus Körperflüssigkeiten durch eine Immunreaktion mit Gewebe-Transglutaminase (tTG), deren immunreaktiven Sequenzen oder Analoga nachgewiesen werden. Gegenstand der Erfindung ist auch die Verwendung von tTG und den genannten Substanzen zur Diagnose und Therapiekontrolle der Sprue oder Zöliakie. Gegenstand der Erfindung ist auch ein orales pharmazeutisches Mittel, das tTG, deren immunreaktive Sequenzen oder deren Analoga als Wirkstoff enthält und zur Behandlung von Sprue oder Zöliakie eingesetzt werden kann, da durch die orale Gabe der genannten Verbindungen eine Immuntoleranz erzeugt wird.
Die vorliegende Erfindung basiert auf der Entdeckung, daß die Gewebe-Transglutaminase (tTG, EC 2.3.2.13) das Autoantigen der Sprue bzw. der Zöliakie ist.

Auf der Basis dieser Erkenntnis wurde das erfindungsgemäße immunologische Verfahren zum Nachweis von Antikörpern gegen tTG entwickelt.

Die Zöliakie ist eine Erkrankung der Dünndarmschleimhaut mit Erstmanifestation vorwiegend im späten Säuglings- und Kleinkindalter. Tritt das entsprechende Krankheitsbild erst beim Erwachsenen auf, so wird sie als einheimische Sprue bezeichnet Beide Begriffe bezeichnen also die gleiche Krankheit. Die Sprue geht mit einer entzündlichen Veränderung der Mukosa und einer dadurch verursachten generalisierten Malabsorption einher. Sie reagiert meist morphologisch und klinisch auf eine Behandlung mit glutenfreier Diät.

Als krankheitsauslösende Faktoren sind die Klebereiweiße (Glutene) von Weizen, Gerste, Roggen und z.T. Hafer bekannt, während die von phylogenetisch weniger verwandten Pflanzenarten wie Mais, Reis und Soja nicht pathogen sind. Unter den Glutenen wird den alkohollöslichen Prolaminen, speziell dem α-Gliadin, die Rolle des krankheitsauslösenden Agens zugeschrieben.
Die Sprue tritt daher bevorzugt in Ländern auf, in denen Weizen als wichtige Nahrungsquelle dient (Europa, USA, Australien) und besitzt z.B. eine Inzidenzrate von 0,14/1000 Neugeborener in Dänemark, 0,7/1000 in Spanien, 1/1000 in Italien, 0,45/1000 in Deutschland und 2,42/1000 in Schweden.
Neuere Untersuchungen belegen jedoch, daß eine subklinische Ausprägung, d.h. eine morphologische Veränderung der Mukosa ohne schwerwiegende Symptome, weit häufiger als bislang vermutet auftritt. So zeigte eine 1994 in Italien durchgeführte Studie eine Inzidenz von 3,28/1000 bei Schulkindern. Das Risiko einer latenten Sprue bei Verwandten 1.Grades von Sprue-Patienten liegt bei bis zu 50 %.

Mit einer vorwiegend latenten Sprue einhergehend tritt gehäuft eine polymorphe Dermatose, die Dermatitis herpetiformis auf, wobei charakteristische subepidermale Bläschen mit granulären IgA-Ablagerungen in den dermalen Papillenspitzen zu beobachten sind. Dünndarmbiopsien zeigen eine unregelmäßige, mehr oder weniger stark geschädigte Mukosa.

Eine weitere gesicherte Assoziation ist zwischen der Sprue und dem Insulin-abhängigen Diabetes mellitus, Schilddrüsenerkrankungen, sowie einer selektiven IgA-Defizienz zu beobachten.
Neben zahlreichen klinischen Begleiterscheinungen der Sprue, wie z.B. einer Anämie, die u.a. einer Vitamin B₁₂-Malabsorption zugeschrieben wird und einem Vitamin K-Mangel, auf den eine erhöhte Blutungsneigung zurückzuführen ist, spielt das stark erhöhte Risiko gastrointestinaler Malignome eine besondere Rolle. Bis zu 15 % der Sprue-Patienten entwickeln, meist im Alter über 50 Jahren, neoplastische Erkrankungen, von denen etwa 50 % auf intestinale T-Zell-Lymphome und weitere 25 % auf Ösophagus-, Oropharynx- und Dünndarm Tumore entfallen.

Die Therapie der Sprue besteht in der strikten Einhaltung einer lebenslangen glutenfreien Diät, wobei nicht nur Gluten enthaltende Produkte aus Weizen, sondern auch aus Roggen, Gerste und Hafer ausgeschlossen werden müssen. Dies bedeutet für die Patienten gravierende Einschränkungen sowohl der Essensgewohnheiten als auch der sozialen Interaktionen.

Sofern die Sprue rechtzeitig diagnostiziert und therapiert wird, besitzt sie eine gute Prognose. Jedoch sind aufgetretene Komplikationen häufig nicht gänzlich reversibel. Wird die Krankheit dagegen nicht erkannt und behandelt, so kann es durch Malabsorption zu schwerwiegenden Krankheitserscheinungen kommen. Letztendlich besteht das erhöhte Risiko einer Entwicklung eines intestinalen Lymphoms, sowie anderer gastrointestinaler Neoplasien.

Für die Diagnose der Sprue und der Verlaufskontrolle unter glutenfreier Diät ist gegenwärtig die Dünndarm-Biopsie der Goldstandard. Zunehmend gewinnen aber auch nicht-invasive Methoden der Diagnostik an Bedeutung, die auf immunologischen Markern beruhen. Da in den Seren der Sprue-Patienten Antikörper der IgA- und der IgG-Klasse vorkommen, die zum einen gegen Gliadin gerichtet sind und zum anderen gegen ein Autoantigen des Endomysiums, einem speziellen Bindegewebe, das u. a. die Kollagene I, III und V, elastische Fasern, nichtkollagene Proteine wie z. B. Fibronektin und Proteoglykane enthält, können die Seren im ELISA auf IgG- und IgA-Antikörper gegen Gliadin, sowie durch indirekte Immunfluoreszenz auf IgG- und IgA-Antikörper gegen Endomysium getestet werden. Während Antikörper gegen Gliadin nicht spezifisch genug für die Sprue sind, wird für die IgA-AK gegen Endomysium eine hohe Sensitivität und Spezifität (97-100 %) berichtet. Für den Immunfluoreszenz-Nachweis werden jedoch Ösophagusschnitte von Primaten benötigt (Dig. Dis. Sci. 1991, S. 752-756; Arch. Dis. In Childhood 1991, S. 941-947; Arch. Dermatol. 1986, S. 459-462). Gegenwärtig gibt es Versuche, die Endomysium-Antikörper auch auf Nabelschnurmaterial nachzuweisen (Dig. Dis. Sci 1995, Abstract). DE 195 20 480 schlägt den Einsatz von aus Affendünndarm, Rattenleber und Schafslungen isolierten Antigengemischen zur enzymimmunometrischen Diagnose der Zöliakie vor.

Ein 90 kDa großes Mannose-reiches Glykoprotein (mit ca. 20% Zuckeranteil), das als Bestandteil in der normalen Haut und der Dünndarmmukosa vorliegt, wurde 1984 in Lancet 1984, 20, S. 892-894 und in Journal of Immunol. Methods 1984, S. 327-336 beschrieben. Bei 10 von 20 Patienten mit Sprue/Zöliakie und 7 von 12 Patienten mit Dermatitis herpetiformis (einer Erkrankung, welche als Manifestation der Sprue in der Haut angesehen wird) konnten zirkulierende IgG-Immunkomplexe nachgewiesen werden, die dieses Glykoprotein erkennen. Das beschriebene 90 kDa-Glykoprotein ist durch einen 20%-Gehalt an Mannoseresten charakterisiert, während die tTG trotz 6 potentieller Glykosylierungsstellen nichtglykosyliert vorliegt (Ichinose et al., J. Biol. Chem. 1990, 265, 13411-13414).

1986 wurden von Maury et al., in GUT 1986, 27, 147-152 diese Antikörper in einem ELISA in Seren von Patienten mit Sprue sowie in Kontrollseren untersucht Hierbei zeigten die Sprue-Patienten im Vergleich zu Patienten mit chronisch entzündlichen Darmerkrankungen, rheumatoiden Erkrankungen oder Kontrollpersonen, einen signifikant höheren Antikörper-Titer (p<0,001), der nach Einhalten einer Gluten-freien Diät auf Normalwerte absank. Eine Korrelation dieser Antikörper mit Retikulinantikörpern bestand nicht.

Im Abstract von W. Dieterich, Gut 1995, Vol. 37, Suppl. 2, A1-A284 wurden ein 90 kDa-Protein und ein 300 kDa-Protein beschrieben, die durch Immunpräzipation erhalten wurden und von denen vermutet wurde, dass es sich um Autoantigene der Sprue handelt.

Bei rechtzeitiger Diagnose und konsequenter Einhaltung einer glutenfreien Diät kann die Erkrankung in Remission gehalten und damit auch das erhöhte Malignom-Risiko der Patienten auf den Normalwert gesenkt werden. Es ist folglich von großem Interesse, einen geeigneten Nachweistest für die Sprue zu entwickeln. Da der Personenkreis mit einer latenten Sprue ebenfalls zur Risikogruppe gehört, sollten alle in Betracht kommenden Personen (vor allem Verwandte 1. Grades), letztendlich alle Schulkinder, wie dies in Italien zur Zeit erwogen wird, mit einem sensitiven, spezifischen, leicht durchführbaren und preiswerten Test untersucht werden.
Große Screening-Programme scheiterten bisher jedoch an folgenden Problemen:
- Die invasiven Duodenal-Biopsien symptomfreier Personen sind unzumutbar und viel zu aufwendig.
- Ein auf Antikörpern gegen Gliadin beruhender ELISA-Nachweis ist aufgrund seiner geringen Spezifität kaum brauchbar.
- Der auf Primaten-Ösophagus basierende Immunfluoreszenz-Nachweis von Endomysium- Antikörpern der IgA-Klasse ist als generelle Screeningmethode zu aufwendig. Ferner ist die Beurteilung subjektiv und erlaubt nicht die Erfassung von Sprue-Patienten mit einer IgA-Defizienz (2 % der Patienten).

Es existiert also bisher kein nicht-invasiver, spezifischer, quantitativer, schnell, leicht und kostengünstig durchzuführender Nachweistest für die Sprue/Zöliakie und deren Therapie-Kontrolle.

Dieses Problem wird durch die vorliegende Erfindung gelöst. Basierend auf der überraschenden Erkenntnis, daß die Gewebe-Transglutaminase (tTG, EC 2.3.2.13) das Autoantigen der Sprue ist, wurde ein immunologisches Verfahren zur Diagnose oder Therapiekontrolle der Sprue oder Zöliakie gemäß der Ansprüche 1 bis 6 entwickelt, bei dem Antikörper gegen tTG aus Körperflüssigkeiten, insbesondere aus dem Serum, nachgewiesen werden durch eine Immunreaktion mit tTG, deren immunreaktiven Sequenzen oder Analoga, wobei die Immunreaktion nicht mit einem Gewebeschnitt eines tierischen oder menschlichen Gewebes durchgeführt wird.

Die Gewebe-Transglutaminase gehört zur Klasse der Transglutaminasen. Die TGn (EC 2.3.2.13) sind Enzyme, die Ca²⁺-abhängig einen Acyltransfer katalysieren, wobei die γ-Carboxamidgruppen von Peptid-gebundenen Glutaminresten als Acyl-Donoren agieren. Als Acyl-Akzeptoren dienen primär proteingebundene Lysinreste, so daß der Transfer in einer ε-(γ-Glutamyl-) Lysin-Bindung resultiert. Die Substratspezifität der TGn bezüglich der Acyl-Donoren ist sehr hoch (Abhängigkeit von der Aminosäure-Sequenz), wohingegen ein außergewöhnlich breites Spektrum an Akzeptoren zur Verfügung steht. Die entstandenen kovalenten Peptidbindungen sind sehr stabil und proteaseresistent, wodurch sich eine erhöhte Beständigkeit der vernetzten Proteine gegenüber chemischen, enzymatischen oder physikalischen Einflüsse ergibt.
Mit dem weitverbreiteten Vorkommen verschiedener TGn in diversen Organen, Geweben, im Plasma und in interstitiellen Körperflüssigkeiten korreliert auch das Vorkommen von durch Transglutaminase modifizierten Proteinen im Blutgerinnsel, auf Zellmembranen, in der Hornschicht der Epidermis, in Haaren, Nägeln und in der extrazellulären Matrix.
Die beschriebenen Transglutaminasen lassen sich durch ihre physikalischen Eigenschaften, ihre Lokalisation im Körper und ihre Primärstruktur unterscheiden.

Die Gewebe-TG (tTG) wird auch zelluläre, Erythrozyten-, endotheliale, zytoplasmatische, Leber- oder Typ II-TG genannt und ist ein Monomer mit einem Molekulargewicht von 75-85 kDa.
Die komplette Aminosäuresequenz mit 687 Resten wurde von der cDNA abgeleitet. Auf Protein-Ebene besteht eine 84 %ige Homologie zwischen dem menschlichen Enzym und dem Enzym aus M ausmakrophagen, sowie eine 81 %ige Homologie zwischen dem menschlichen und dem Meerschweinchen-Enzym. Nukleotidaustausche zwischen den Spezies sind oftmals ohne Auswirkung auf die Aminosäuresequenz. Stark konserviert ist das aktive Zentrum mit einer ausgeprägten Protein-Homologie zwischen den drei Spezies (49 von 51 Resten sind identisch) und einem hohen Grad an Protein-Homologie (75 %) zur a-Untereinheit des Faktor XIII (vgl. Gentile, V. et al., J. Biol. Chem. 1991; 266: 478-483; Greenberg, C.S. et al., FASEB J. 1991; 5: 3071-3077).
Es liegen weder ein Signalpeptid noch eine Glykosylierung vor, und trotz mehrerer Cysteinreste existieren offenbar keine Disulfidbrücken. Fluoreszenz-Hybridisierungen lokalisierten das Gen für die humane Gewebe-Transglutaminase auf dem Chromosom 20q12 (Gentile, V. et al., Genomics 1994; 20: 295-297). Obwohl der Mechanismus der Enzym-Ausschleusung noch unklar ist, gibt es eindeutige Belege, daß die intrazellulär ubiquitär vorkommende tTG in der extrazellulären Matrix (EZM) wichtige Aufgaben übernimmt. Zudem wird die für die Aktivität der tTG erforderliche Ca²⁺-Konzentration unter physiologischen Bedingungen intrazellulär kaum erreicht, während im Extrazellulärraum dagegen ausreichend hohe Ca²⁺-Konzentrationen vorliegen (Gentile, V. et al., J. Cell Biol. 1992; 119: 463-474).
Mehrere Untersuchungen belegen eine Assoziation von tTG mit dem EZM-Protein Fibronektin. Neben Fibronektin konnten auch die EZM-Moleküle Nidogen, das N-terminale Prokollagen III-Peptid, die Kollagene V und XI, Osteonectin, ein Mikrofibrillen-assoziiertes Glykoprotein, hochmolekulares Dermatan Sulfat-Proteoglykan und das Lektin Galectin 3 als spezifische Substrate für die tTG identifiziert werden.
Hinweise für eine wichtige Rolle der tTG an der Wundheilung kamen u.a. von Immunfluoreszenz-Studien an kultivierten WI38-Zellen (embryonalen Lungenfibroblasten), die unter Normalbedingungen keine extrazelluläre tTG-Aktivität aufweisen, das Enzym jedoch nach künstlicher Wundsetzung extrazellulär ablagern. Dem möglicherweise passiven Austritt des Enzyms aus geschädigten Zellen schließt sich eine zunächst nicht-kovalente Bindung an die EZM, insbesondere an Fibronektin und fibrilläre Kollagene an. Dort ist das Enzym einige Stunden katalytisch aktiv (Upchurch, H.F. et al., J. Cell. Physiol. 1991; 149: 375-382). Am Rattenmodell wurde, ebenfalls nach künstlicher Wundsetzung, 5 Tage lang eine erhöhte tTG-Aktivität nachgewiesen (Bowness, J.M. et al., Biochim. Biophys. Acta. 1988; 967: 234-240). Auch bei der Inkubation von humanen Erythrozytenlysaten mit Plasma konnte eine starke Affinität der freigesetzten tTG an Fibronektin demonstriert werden (Lorand, L. et al., Proc. Natl.

Acad. Sci. USA. 1988; 85: 1057-1059). Alle Befunde deuten darauf hin, daß die an die EZM gebundene tTG eine zentrale Rolle in der frühen Phase der Wundheilung übernimmt, insbes. zusammen mit Faktor XIIIa bei der Fibrin-Stabilisierung mitwirkt und durch ein Quervemetzen von extrazellulären Proteinen eine Schutzschicht und ein stabiles adhäsives Substrat um die geschädigten Zellen bildet. Bisher konnten in Vertebraten keine Enzyme nachgewiesen werden, die in der Lage sind, die von der tTG katalysierten, überaus stabilen Quervernetzungen der Proteine zu spalten.

Basierend auf der Erkenntnis, daß die tTG das Autoantigen der Sprue ist, ist auch die Verwendung von tTG, deren immunreaktiven Sequenzen oder Analoga zur Diagnose und Therapiekontrolle der Sprue oder Zöliakie Gegenstand der Erfindung, wobei kein Gewebeschnitt eines tierischen oder menschlichen Gewebes verwendet wird. Da sich der Test schnell und kostengünstig durchführen läßt, ermöglicht er ein effizientes Screening der Bevölkerung auf tTG-Antikörper.

Die erfindungsgemäß eingesetzte tTG kann humanen, tierischen, synthetischen oder rekombinanten Ursprungs sein. Als tTG-Analoga werden im Sinne der Erfindung alle antigenen Strukturen verstanden, die mit Antikörpern gegen tTG eine immunreaktion eingehen, z.B. synthetische Peptide. Als immunreaktive Sequenzen sind proteolytisch, synthetisch oder gentechnisch hergestellte Fragmente der tTG und deren durch Austauch von Aminosäuren erhaltene Varianten zu verstehen.

Der erfindungsgemäße immunologische Nachweis wird mittels bekannter Methoden durchgeführt. So kann zur Detektion der Patientenantikörper jedes beliebige direkte (z.B. mit einem Sensorchip) oder indirekte Verfahren zur Anwendung kommen.

Bei den direkten Verfahren wird die Bindung der nachzuweisenden Antikörper an das Antigen über eine Änderung der chemischen oder physikalischen Eigenschaften bestimmt, so daß nachfolgende Detektionsschritte mit markierten Bindungspartnern nicht notwendig sind.

Erfindungsgemäß bevorzugt erfolgt der Nachweis der tTG-Antikörper in einem Immunoassay, bevorzugt in einem Festphasenimmunoassay, unter direkter oder indirekter Kopphmg eines Reaktionspartners mit einer gut nachweisbaren Markierungssubstanz. Besonders bevorzugt kann der Nachweis in einem ELISA, einem RIA oder einem Fluoreszenzimmunoassay erfolgen. Die Durchführung dieser Nachweisverfahren ist dem Fachmann gut bekannt.

So wird z.B. in einem ELISA das Antigen, im vorliegenden Fall z.B. tTG, direkt oder indirekt an eine Trägersubstanz, wie beispielsweise Polystyrol, gebunden. Nach Inkubation mit den nachzuweisenden Antikörpern, z.B. aus dem Serum von Patienten, werden Antigen-gebundene Antikörper direkt oder indirekt mittels Enzym-gekoppelter Substanzen nachgewiesen. Diese Substanzen können Antikörper, Fragmente von Antikörpern oder hochaffine Liganden wie z.B. Avidin, das an eine Biotin-Markierung bindet, sein. Als Enzyme kommen beispielsweise die Peroxidase, alkalische Phosphatase, β-Galaktosidase, Urease oder Glucoseoxidase in Betracht. Durch Zugabe eines chromogenen Substrats können die gebundenen Enzyme und damit beispielsweise die gebundenen tTG-Antikörper quantifiziert werden.

Auch in einem Radio-Immunoassay ist das Antigen, z.B. die tTG, direkt oder indirekt an eine Trägersubstanz, wie beispielsweise Polystyrol, gebunden. Nach Inkubation mit den nachzuweisenden Antikörpern, z.B. aus dem Serum von Patienten, werden Antigen-gebundene Antikörper mittels Substanzen nachgewiesen, die eine radioaktive Markierung, beispielsweise ¹²⁵I, tragen. Diese Substanzen können Antikörper, Fragmente von Antikörpern oder hochaffine Liganden wie z.B. Avidin, das an eine Biotin-Markierung bindet, sein. Die gebundene Radioaktivität kann mittels eines geeigneten Meßgeräts quantifiziert werden.

Nach dem gleichen Prinzip werden in einem Fluoreszenzimmunoassay die Antigengebundenen Antikörper mittels Substanzen nachgewiesen, die eine Fluoreszenz-Markierung, beispielsweise Fluorescein-Isothiocyanat (FITC) tragen. Diese Substanzen können Antikörper, Fragmente von Antikörpern oder hochaffine Liganden wie z.B. Avidin, das an eine Biotin-Markierung bindet, sein. Die gebundene Menge an Fluoreszenzfarbstoff wird dann mittels eines geeigneten Meßgeräts quantifiziert.

Erfindungsgemäß ist es auch möglich, die Patientenantikörper in einem Agglutinationstest oder Geldiffusionstest nachzuweisen. Auch diese Nachweisverfahren sind dem Fachmann bekannt. So werden beim Geldiffusionstest in benachbarte, naheliegende Vertiefungen von Agar- oder Agarose-Platten z.B. die Antigen- bzw. die Antikörper- Lösungen gefüllt. Im vorliegenden Fall kann die Antigenlösung beispielsweise die tTG-Lösung und die Antikörperlösung beispielsweise Blutserum sein. Diffundieren die Substanzen aus ihren Vertiefungen, bilden sich ausgehend von den Vertiefungen Konzentrationsgradienten. Wenn die überlappenden Antigen- und Antikörper-Konzentrationen im Gel innerhalb bestimmter Verhältnisse liegen und die Antikörperlösung Antikörper gegen das Antigen enthält, bilden sich im Gel sichtbare Präzipitate.

Beim Agglutinationstest werden Antigen (z.B. tTG)- tragende Partikel, z.B. aus Latex oder Polystyrol durch Antikörper, beispielsweise aus dem Serum, quervernetzt. Die gebildeten Aggregate lassen sich beispielsweise turbidimetrisch nachweisen.

Erfindungsgemäß ganz besonders bevorzugt wird der Nachweis mit einem IgA-spezifischen oder IgG-spezifischen ELISA im Serum von Sprue-Patienten durchgeführt. Dabei hat sich gezeigt, daß sich der auf der tTG basierende neu entwickelte ELISA-Nachweis von IgA-Antikörpern im Serum von Sprue-Patienten aufgrund seiner hohen Sensitivität und Spezifität hervorragend zur Diagnostik und Therapie-Kontrolle der Sprue eignet. Dies wird auch bei der Verlaufs-Kontrolle der behandelten Patienten deutlich (Titer-Abfall unter Therapie). Ein Vergleich der erfindungsgemäßen ELISA-Daten mit den Immunfluoreszenz-Auswertungen Dritter (Nachweis von IgA anti-Endomysium) zeigt eine gute Übereinstimmung. Unstimmigkeiten treten vor allem bei niedrigen Antikörper-Titern auf, was jedoch zu Lasten der bisher als Goldstandard geltenden indirekten Immunflunreszenz geht. Dies ist u.a. bedingt durch die subjektive Auswertung und die unspezifischen Begleitreaktionen dieser Methode des Standes der Technik.
Der entsprechende, auf Antikörpern anderer Klassen beruhende Nachweis, aufgezeigt am Beispiel der IgG-Antikörper, eignet sich zum Auffinden von Sprue-Patienten mit einer IgA-Defizienz.
Eine weitere Verbesserung der Nachweismethode tritt bei Verwendung aufgereinigter tTG aus Meerschweinchen, humaner tTG, proteolytisch oder gentechnisch erhaltener immunreaktiver Sequenzen oder Analoga sowie synthetischer immunogener tTG-Peptide im Testsystem ein.

Gegenstand der Erfindung ist weiterhin ein orales pharmazeutisches Mittel zur Behandlung von Erkrankungen, die mit einer Immunreaktion gegen tTG, deren immunreaktive Sequenzen oder Analoga einhergehen, gemäß Anspruch 8. Vorzugsweise ist die orale Darreichungsform eine Tablette oder Kapsel. Dabei wird durch Verabreichung von tTG, deren immunreaktiven Sequenzen oder Analoga eine orale Toleranz erzeugt. Die orale Toleranz wird zum einen durch die orale Zufuhr des Autoantigens erreicht, zum anderen existiert ein sog. "Bystander-Effekt": sofern das die Krankheit auslösende Autoantigen nicht bekannt ist, kann in einigen Fällen ein anderes Antigen, das im Zielorgan mit dem Immunsystem in Kontakt kommt, zur oralen Therapie verwendet werden. Dieses Antigen ist dann in der Lage, lokal die Antigen-spezifischen Suppressor-T-Zellen zu stimulieren und dadurch eine systemische Immunantwort zu unterdrücken. Erst bei höherer Antigen-Dosis wird eine Anergie autoreaktiver T-Zellen ausgelöst.

Die orale Toleranz ist die praktische Behandlungsmethode von diversen Autoimmunerkrankungen.

Bevorzugt dient das erfindungsgemäße pharmazeutische Mittel zur Behandlung der Sprue.

Erfindungsgemäß werden die tTG, deren immunreaktive Sequenzen oder Analoga in einer Dosis 0,01-100mg/kg Körpergewicht gegeben.

Das erfindungsgemäße pharmazeutische Mittel kann gegebenenfalls pharmazeutisch verträgliche Hilfsstoffe enthalten, wie z. B. in der Galenik übliche Füllstoffe, Gleitmittel, Sprengmittel, Bindemittel oder Formentrennmittel. Der Anteil der pharmazeutischen Hilfsstoffe kann in Abhängigkeit vom gewählten Wirkstoffgehalt in weiten Grenzen variieren und beträgt jeweils 0,1 bis 20 Gew.%.

Die mit der vorliegenden Erfindung erreichten Vorteile bestehen sonst insbesondere in einem nicht-invasiven, hoch spezifischen, gegen das direkt mit der Erkrankung assoziierte Agens gerichteten Nachweistest für die Sprue und deren Therapie-Kontrolle. Darüberhinaus besteht der große Vorteil des entwickelten Tests in der schnellen, leicht und kostengünstigen Durchführbarkeit und der Standardisierbarkeit zwischen verschiedenen Labors. Der Test ermöglicht dadurch ein effizientes Screening der Bevölkerung auf Antikörper, gerichtet gegen die tTG.
Die Möglichkeit der quantitativen Auswertung der Test-Daten ist zudem durch deren Objektivität gegenüber der subjektiv geprägten Auswertung einer Immunfluoreszenz überlegen. Immunfluoreszenz-Auswertungen werden außerdem, vor allem bei niedrigen Titern, durch unspezifische Begleitreaktionen erschwert. Durch den Einsatz des spezifischen Autoantigens im Testsystem können die bei der Immunfluoreszenz auf Ösophagusmaterial von Primaten bzw. Nabelschnüren unspezifischen Reaktionen weitgehendst ausgeschaltet werden.

Da der Test sowohl auf Antikörper der IgA- als auch anderer Antikörper-Klassen anwendbar ist, werden auch Sprue-Patienten mit einer IgA-Defizienz erfasst.

Desweiteren besteht durch die Identifizierung der tTG als Autoantigen der Sprue die Möglichkeit, dieses in seiner Gesamtheit bzw. dessen immunreaktive Epitope (proteolytisch oder gentechnisch hergestellte Sequenzen, Analoga oder synthetische Peptide) zur oralen Therapie der Sprue einzusetzen.

### Ausführungsbeispiele

### Beispiel 1

### Isolierung und Charakterisierung des Autoantigens

### 1.1 Immunfluoreszenz, APAAP-Färbungen

Die Färbungen wurden auf verschiedenen, in 100 % Methanol über 2 min bei -20 °C fixierten Zellinien, durchgeführt.
Beim Immunfluoreszenz-Nachweis wurden die Präparate mit Sprue- bzw. Kontrollseren inkubiert, gewaschen und mit einem TRITC-markierten anti-human-IgA aus Kaninchen detektiert (Schuppan et al., J. Biol. Chem. 1990;265:8823-32).
APAAP-Markierungen wurden nach Inkubation der Zellen mit den Sprue-Seren, Waschen und der darauffolgenden Detektion mit dem APAAP-Komplex durchgeführt (Cordell J. L. et al., J. Histochem. Cytochem. 1984;32:219-229)
Dabei zeigten HT1080 (humane Fibrosarkom-Zellen), WI38 (humane embryonale Lungenfibroblasten), Hep1 und HepG2 (Hepatokarzinom-Zellen) mit den Patientenseren eindeutig positive, zytoplasmatische Signale, wogegen Normalseren oder auch eine Vorbehandlung mit humanem IgA keine Markierung zeigten. Humane Vorhautfibroblasten, humane Rhabdomyosarkom (RD)- / Ratten-Ito- / Ratten-Morris-Hepatom- und Hund-MDCK-Zellen zeigten nur sehr schwache bis negative Reaktionen.

### 1.2. Metabolische Zellmarkierung und Immunpräzipitation des Autoantigens

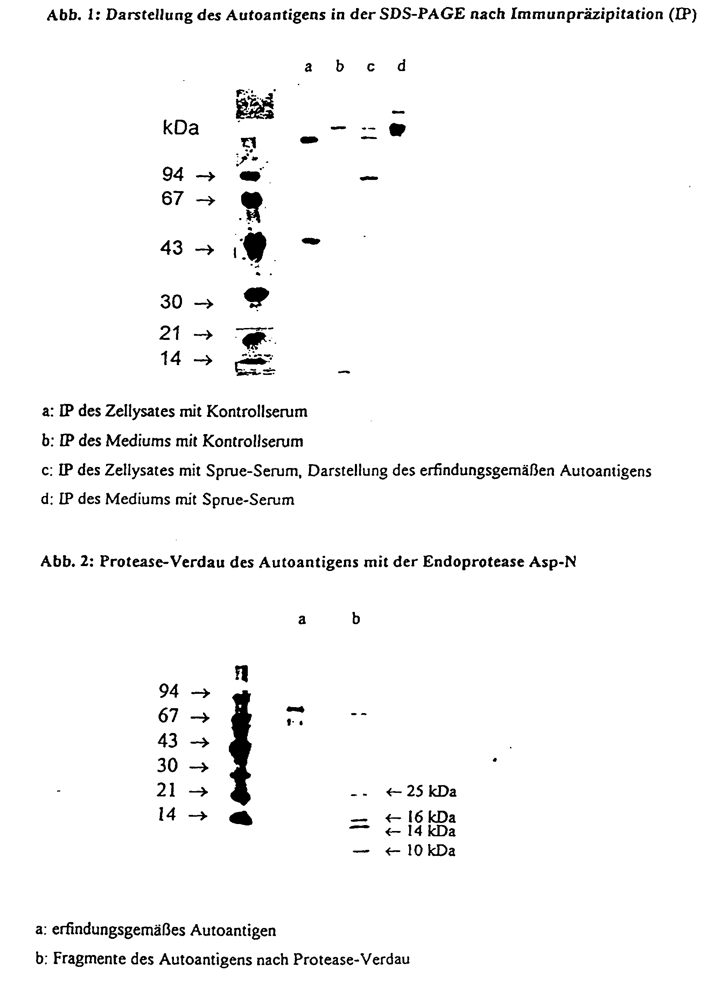

Die Charakterisierung und Isolierung des Autoantigens wurde aus HT1080 Zellen durchgeführt.
Die Zellen wurden in Dulbecco's modifiziertem Eagle Medium (DMEM, Gibco) mit L-Alanyl-L-Glutamin, 10 % fötalem Kälberserum (FKS, Gibco), 100 U/ml Penizillin und 100 µg/ml Streptomyzin (Seromed) bei 37 °C und 8% CO₂ kultiviert. Zur metabolischen Markierung wurden die Zellen in Kulturschalen mit 5 cm Durchmesser überführt und bei Erreichen einer ~90 %-igen Konfluenz 2 h in Methionin- und FKS-freiem Medium gehalten, bevor dieses gegen 3 ml FKS-freies, ³⁵S- Methionin (0,2 mCi, Expre³⁵S³⁵S, NEN-Dupont) enthaltendes Medium ausgetauscht wurde. Nach 16-20-stündiger Inkubation wurde der Überstand abgenommen. Die Zellen wurden mit Phosphatpuffer (PBS, Seromed) gespült und anschließend in 3 ml Lysepuffer (50 mM Tris+HCl, 150 mM NaCl, 0,5 % Triton X-100, 0,5 % nichtionisches Detergenz IGEPAL CA-630 [Sigma], Proteaseinhibitor Complete®[Boehringer], pH 7,5) lysiert. Im Anschluß wurde sowohl mit dem Medium als auch dem Zellysat eine Immunpräzipitation mit CNBr-aktivierter Sepharose 4B (Pharmacia) durchgeführt.
Die Aktivierung und Bindung an die Sepharose erfolgte nach Herstellerangaben. Nach dem Quellen und Waschen in 1 mM HCl, pH 2,5 wurde die CNBr-aktivierte Sepharose in 0,1 M NaHCO₃, 0,5 M NaCl, pH 8,3 , mit einem gegen humanes IgA gerichteten Antikörper aus Kaninchen (Dianova, 2,4 mg Antikörper/ml Sepharose) über Nacht bei 4 °C inkubiert. Nicht gebundene Antikörper wurden durch Waschen mit dem Bindungspuffer entfernt, nicht besetzte Bindungsstellen durch Zugabe von 1 M Ethanolamin, pH 9,0, bei Raumtemperatur 2 h abgesättigt. Danach wurde die Sepharose 3 x alternierend (je 10 x Vol.) mit 0,1 M Natriumacetat, 0,5 M NaCl, pH 4,0, und 0,1 M Tris-HCl, 0,5 M NaCl, pH 8,0 , gespült. Es folgte eine Inkubation der Sepharose mit Seren von Sprue-Patienten bzw. gesunden Personen (0,5 ml Serum/ml Sepharose) bei 4 °C über Nacht in Bindungspuffer (50 mM Tris-HCl, 150 mM NaCl, 1 mM CaCl₂, 1 mM MgCl₂, pH 8,0). Überschüssige Serum-Antikörper wurden durch 3 x Spülen mit Bindungspuffer entfernt.
Je 1 ml HT1080-Medium oder Zellysat (ca 5x10⁴ Zellen) der metabolisch markierten Zellen wurden 30 min bei Raumtemperatur mit 50 µl C14B-Sepharose (Pharmacia) vorinkubiert, um unspezifisch bindende Proteine zu entfernen. Nach Abzentrifugieren (10 000 x g, 5 min, 4 °C) wurden die Überstände mit je 50 µl der Sepharose, an die zuvor IgA der Patienten bzw. Kontrollpersonen gebunden wurde, über Nacht unter Schütteln bei 4 °C inkubiert. Dann wurden die Sepharose-Pellets je 3 x mit 1 ml Waschpuffer (10 mM Tris-HCl, 1 % IGEPAL CA-630 [Sigma], 0,5 % Natriumdesoxycholat, 0,1 % Natriumlaurylsulfat, Complete®[Boehringer], pH 8,0) gewaschen, gefolgt von 1 ml 10 mM Tris-HCl, pH 8,0. Danach wurden die Pellets in SDS-Probenpuffer aufgenommen, bei 95 °C 5 min unter reduzierenden oder nicht reduzierenden Bedingungen inkubiert, im 10-12,5% SDS-Polyacrylamidgel aufgetrennt (Lämmli, U.K, Nature 1970;227:680-685) und in der Autoradiographie nachgewiesen (**Abb.1**).

Das gebundene hochmolekulare Protein aus dem Medium erwies sich bei weiteren Untersuchungen als Fibronektin, welches u.a. unspezifisch an die Sepharose gebunden wird.
Eine zellassoziiertes Protein von 85 kDa konnte jedoch mit allen 30 bisher so eingesetzten Sprue-Seren präzipitiert werden, während dies mit 15 Kontrollseren, darunter Normalseren, Seren von Patienten mit Colitis Ulcerosa und Sjögrens-Syndrom, nicht möglich war. Hieraus wurde gefolgert, daß dieses Protein das wesentliche Autoantigen der Sprue repräsentiert.
Der autoradiographisch sichtbaren 85 kDa-Bande wurde in einer Proteinfarbung der Gele mit Silbernitrat (Heukeshoven, J., et al., Electrophoresis 1985;6:103-112) eine scharfe Proteinbande zugewiesen.

### 1.3. Isolierung und Aufreinigung des 85 kDa Autoantigens

Zur Isolierung größerer Mengen des Autoantigens wurden insgesamt 65 Kultuschalen (je 175 cm²) der HT1080 Zellen (etwa 10⁹ Zellen) kultiviert. Kurz vor Erreichen der Konfluenz wurde das Medium gegen FKS-freies Medium ausgetauscht, gefolgt von einer Inkubation über weitere 16-20 h im CO₂-Inkubator. Die Lyse bzw. Immunpräzipitation erfolgten wie oben beschrieben. Das Sepharosepellet wurde in insgesamt 4,5 ml SDS-Probenpuffer mit 2 % DL-Dithiothreitol (Sigma) 5 min bei 95 °C inkubiert, um gebundene Proteine zu lösen und anschließend im analytischen SDS-Polyacrylamidgel überprüft.
Zur weiteren Aufreinigung des Autoantigens wurde das Immunpräzipitat über eine Elutionselektrophorese mit einer Prep Cell (Model 491 BIO-RAD) wie folgt aufgetrennt: Auf ein Randgel (Außen-Durchmesser 3 cm), bestehend aus 6,5 cm Trenngel (8 % Polyacrylamid, pH 8,8) und 1,5 cm Sammelgel (4 % Polyacrylamid, pH 6,8) wurden die 4,5 ml des Proteingemisches aufgetragen und elektrophoretisch aufgetrennt. Die einzelnen Proteine wurden im Elutionspuffer (25 mM Tris-HCl, 0,1 M Glycin, 0,01 % SDS, pH 8,3) in Fraktionen à 1,2 ml (0,8 ml/min) gesammelt. Die eluierten Fraktionen wurden in der SDS-PAGE kontrolliert und die das gewünschte Protein enthaltenden Fraktionen (etwa 15 ml) vereinigt und mit Hilfe einer Ultrafiltration (Amicon Centriprep- 50 bei 1000 x g) auf circa 1 ml Gesamtvolumen aufkonzentriert.

### 1.4. Protease-Verdau des Autoantigens

Unter mehreren getesteten Proteasen wurde die Endoproteinase Asp-N (sequencing grade, Boehringer Mannheim) als zur Fragmentierung geeignet ermittelt, da sie ein weitestgehend reproduzierbares Spaltmuster mit relativ gut trennbaren Fragmenten ermöglichte. Die Enzym- zu Substratkonzentration wurde auf 1:100 eingestellt und der Verdau über 30 min bei 37 °C durchgeführt.

### 1.5. Transfer auf PDVF-Membran

Nach Verdau des aufgereinigten Autoantigens wurden die Peptidfragmente auf einem präparativen 10 % Tricine-GeI aufgetrennt (Schägger, H. et al., Anal Biochem. 1987;166:368-379) (**Abb.2**) und bei 4 °C im Semi-Dry-Fastblot-Verfahren unter Verwendung graphithaltiger Elektrodenplatten (Fastblot B32/33, Biometra) auf eine PVDF-Membran (Polyvinylidendifluorid, Immobilon™, Millipore) transferiert. Dazu wurden folgende Schichten auf die Anodenplatte gelegt: 1.) ein Filterpapier, getränkt in Anodenpuffer 1 (300 mM Tris-HCl, 20 % Methanol, pH 10,4), 2.) ein Filterpapier, getränkt in Anodenpuffer 2 (30 mM Tris-HCl, 20 % Methanol, pH 10,4), 3.) die PVDF-Membran, aktiviert in Methanol und präequilibriert in Anodenpuffer 2, 4.) das Tricine-Gel, 5.) zwei Filterpapiere, getränkt in Kathodenpuffer (25 mM Tris-HCl, 40 mM ε-Amino-n-Capronsäure, 20 % Methanol, pH 9,4), 6.) die Kathodenplatte. Der Transfer wurde 35 min bei 180 mA durchgeführt.
Die PVDF-Membran wurde daraufhin in 0,1 % Coomassie Blue Serva R-250, 50 % Methanol für 5 min gefärbt, mit 50 % Methanol, 10% Essigsäure entfärbt, gründlich mit destilliertem Wasser gewaschen und luftgetrocknet. Charakteristische Banden des verdauten Autoantigens bei 10 kDa, 14 kDa, 16 kDa und 25 kDa wurden sorgfältig ausgeschnitten und N-terminal ansequenziert.

### 1.6. Edman-Abbau

### (gemäß Edman and Henschen in: Needleman, S.B.: Protein Sequence Determination, Springer Verlag, Berlin. 1975;232-279)

Die Sequenzierung in einem Applied Biosystems 4778-Sequenator ergab drei Aminosäure-Sequenzen, welche mit der Swiss-Prot 31 Datenbank (von PC/GENE, IntelliGenetics) verglichen wurden. Daraus konnte bei minimaler Diskordanz, eine eindeutige Zuordnung der drei Fragmente zur humanen Gewebe-Transglutaminase (t TG, EC 2.3.2.13, Protein-Glutamin Gamma-Glutamyltransferase) gemacht werden; die Angaben erfolgen im one letter code', X bedeutet keine Identifizierung:

t-Transglutaminase:          28´ REKLVVRRGQPFW
10 kDa-Fragment:                REKLVVRRGQPF(S)

t-Transglutaminase: 581' DLYLENPEIKIRILG
14 kDa-Fragment: DLYLENPEIXIXILG

t-Transglutaminase: 438′ DITHTYKYPE
16 KDa-Fragment: DITLTYQYP(V)

Dem 25 kDa-Fragment konnte keine eindeutige Sequenz zugeordnet werden, da es sich dabei um ein Peptidgemisch handelt.

### Beispiel 2

### Bestätigung der Gewebe-Transglutaminase (tTG) als Sprue-Autoantigen

### 2.1. Immunpräzipitation der tTG vom Meerschweinchen

Bei kommerzieller Verfügbarkeit und einer Sequenz-Homologie (>80 %) zur humanen tTG wurde die tTG aus der Leber des Meerschweinchens (Sigma) zunächst gelelektrophoretisch aufgetrennt, um dessen Reinheit zu überprüfen. Die tTG stellt dabei neben mehreren anderen Proteinen mit ca 50 % eine der Hauptbanden dar.

Obwohl sich die humane tTG mit 687 Aminosäuren nur geringfügig vom Meerschweinchenprotein mit 690 Aminosäuren unterscheidet, besitzen die beiden Proteine ein sehr unterschiedliches Laufverhalten im SDS-Polyacrylamidgel. Während das Protein tierischen Ursprungs erwartungsgemäß bei 75-80 kDa erscheint, wandert das humane Protein deutlich weniger schnell und täuscht, wie auch in der Literatur beschrieben, trotz offenbar fehlender N-Glykosylierung ein apparentes Molekulargewicht von 85 kDa vor (Gentile, V., et aL, J. Biol. Chem. 1991;266:478-483)
Die Reaktivität des menschlichen Auto-Antikörpers aus Sprue-Seren mit der Meerschweinchen-tTG wurde in einer Immunpräzipitation getestet. Dazu wurden 4µg tTG (Sigma) in 500µl Lysispuffer, 0,5 % Rinderserumalbumin mit an 4B-Sepharose gekoppeltem Sprue-IgA bei 4 °C über Nacht geschüttelt, gewaschen, in SDS-Probenpuffer unter reduzierenden Bedingungen gekocht und im 10 % Polyacrylamidgel aufgetrennt (s. 4.1.2.). Hier zeigte sich eine spezifische Präzipitation der erwarteten Bande (Mᵣ 80 kDa) nicht aber der Verunreinigung.

### 2.2. Bestätigung der tTG als Autoantigen im Westernblot

Nach Auftrennung von 2 µg der tTG aus Meerschweinchen im SDS-Gel und Transfer aufNitrozellulose wurde der Blot bei 4 °C in PBS, 2 % fettarmen Magermilchpulver, 0,3 % Tween 20, pH 7,3 über Nacht blockiert. Es folgten eine einstündige Inkubation mit Sprue-Serum (1/200) in demselben Puffer, drei Waschschritte und eine einstündige Inkubation mit an alkalischer Phosphatase gekoppelten Antikörpern aus Kaninchen gegen humanes IgA (1/500). Die Blots wurden in PBS gewaschen und mit Nitro Blue Tetrazolium und 5-Brom-4-Chlor-3-Indolylphosphat als Substrat entwickelt (Blake, M.S., et aL, Anal. Biochem. 1984;136:175-179)
Die 75-80 kDa-Bande ergab ein eindeutiges positives Signal mit dem Sprue-Serum, als weiterer Beweis dafür, daß die Seren von Spree-Patienten Antikörper der IgA-Klasse gegen die tTG enthalten, während Kontrollseren kein Signal ergaben.

### 2.3. Bestätigung der tTG als das Endomysium-Autoantigen in der indirekten Immunfluoreszenz

Ösophagus-Gewebeschnitte von Primaten (Euroimmun, Deutschland) wurden zum indirekten Nachweis der IgA-Antikörper gegen Endomysium in Sprue-Seren, bzw. deren Inhibition durch tTG varwendet. Nach Vorinkubation von 10 µl des 1/320 m PBS verdünnten Patientenserums mit 0,5 oder 10 µg tTG aus Meerschweinchen (Sigma) bzw. 10 µg BSA (Sigma) über 1h bei Raumtemperatur, erfolgte dessen Inkubation mit den Ösophagus-Schnitten 1 h bei Raumtem-peratur in feuchter Atmosphäre. Zur Positiv- bzw. Negativ-Kontrolle dienten Sprue-Serum (1/320) bzw. Seren von Gesunden (1/50). Nach dreimaligem Waschen der Schnitte in PBS/ 0,2% BSA und Lufttrocknen wurde die Detektion des Autoantigens mit einem TRITC-markierten, gegen humanes IgA gerichteten, Antikörper aus Kaninchen (Dianova), 1/50 in PBS verdünnt, 1 h bei Raumtemperatur durchgeführt. Überschüssige Antikörper wurden durch sukzessives Waschen mit PBS/0,2 % BSA, PBS und destilliertem Wasser entfernt.
Das Patientenserum zeigte eine deutliche Anfärbung der EZM durch die Antikörper der IgA-Klasse, die durch Zugabe von steigenden Konzentrationen an tTG inhibiert wurden, nicht jedoch durch Vorinkubation mit BSA. Die Kontrolle mit Serum von Gesunden zeigte keinerlei Anfärbung der Ösophagus-Schnitte.

### Beispiel 3:

### 3.1. Entwicklung eines Sprue-spezifischen ELISA mit IgA-Antikörpern zur Diagnostik und Verlaufskontrolle der Sprue

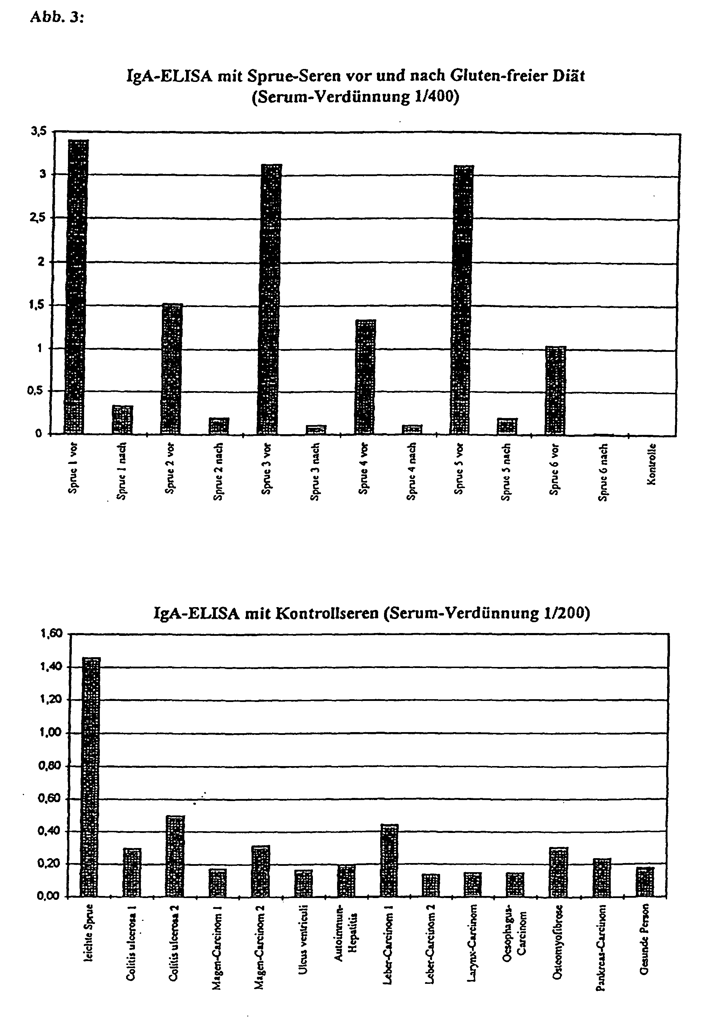

In Polystyrol-Mikroplatten (Greiner Labortechnik, 96 Wells) wurde pro Vertiefimg 1 µg Meerschweinchen-Transglutaminase (Sigma T-5398) in 100µl PBS pipettiert und 2 h bei 37°C unter leicht rotierenden Bewegungen inkubiert. Nicht gebundene tTG wurde durch Spülen mit PBS (3 x 200µl) entfernt, freie Bindungstellen der Vertiefungen wurden mit 1 % Rinderserum-albumin (Sigma) in 250 µl PBS über Nacht bei 4 °C blockiert. Nach Waschen mit PBS/0,1 % Tween-20 (3 x 200 µl) wurden die Vertiefungen mit sequentiellen Serum-Verdünnungen in PBS/0,1 % Tween-20 (100 µl) für 1 h bei Raumtemperatur unter leicht rotierenden Bewegungen inkubiert, mit PBS/0,1 % Tween-20 (3 x 200µl) gewaschen und daraufhin mit einem Peroxidase-konjugierten, gegen humanes IgA gerichteten Antikörper aus Kaninchen (Dianova) (1/400 in 100 µl PBS/0,1 % Tween-20) 1 h bei Raumtemperatur inkubiert. Nach Waschen mit PBS (3 x) erfolgte eine 30 min Inkubation bei Raumtemperatur im Dunkeln mit je 200 µl 0,1M Citratpuffer, 17,6 mM H₂O₂, 5,5 mM o-Phenylendiaminhydrochlorid (Sigma), pH 4,2 und die anschließende Detektion des gebildeten Farbstoffs im ELISA-Reader (MRX, Dynatech Laboratories) bei 450 nm.
Getestet wurden 20 Seren von Sprue-Patienten vor und nach Therapie mit Gluten-freier Diät, d.h. in der aktiven und weniger aktiven Phase der Erkrankung. Das Testsystem erwies sich als hoch sensitiv, mit einer guten Korrelation der Werte zur aktiven Phase der Sprue. Die Therapie-Erfolge durch Einhalten einer Diät spiegeln sich in einer Abnahme der IgA-Antikörper gegen die tTG wider. Die große Spezifität zeigt sich in der geringen Extinktion (Hintergrund-Level) der Kontrollseren von Gesunden, Patienten mit Colitis ulcerosa, Leberzirrhose, diversen Tumoren, Sjögrens Syndrom u.v.m. (**Abb.3**).

### 3.2. Entwicklung einer ELISA mit Antikörpern anderer Klassen zur Diagnostik und Verlaufskontrolle der Sprue am Beispiel der IgG-Antikörper

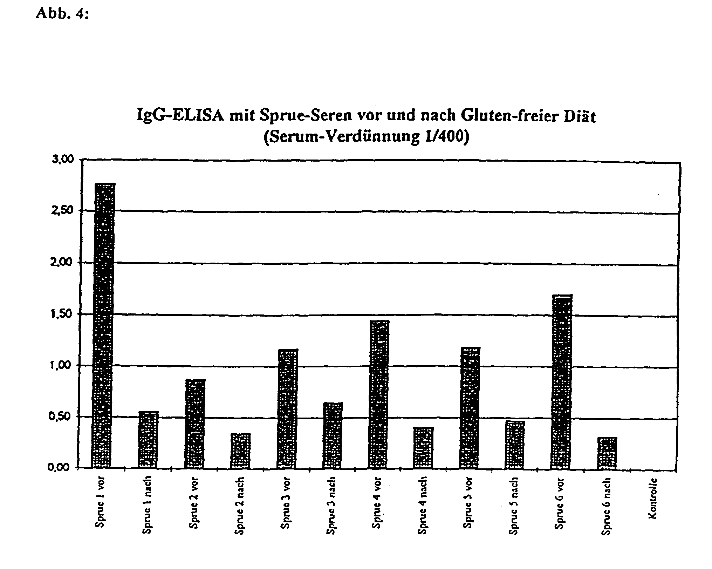

Da ca 2 % der Sprue-Patienten eine IgA-Defizienz besitzen, wurden die Seren auf ihre Sensitivität und Spezifität von IgG-Antikörpern gegen tTG getestet. Die Durchführung des ELISA erfolgte wie unter 3.1. Es wurde lediglich der Peroxidase gekoppelte anti-human IgA Antikörper (Dianova), gegen einen anti-human IgG Antikörper (Dianova) ausgetauscht. Die Werte der Sprue-Patienten entsprachen in ihrer Sensitivität, sowohl vor als auch nach Gluten-freier Diät, den mit IgA Antikörpern erhaltenen Daten.
Einige Kontrollseren zeigten leicht erhöhte Werte, was früheren Befunden einer verminderten Spezifität der Endomysium-Antikörper in der indirekten Immunfluoreszenz der IgG-Klasse entspricht (**Abb.4**).

### Beispiel 4:

### Neue Funktion der Gewebe-Transglutaminase (tTG) in der Quervernetzung von Gliadin

Während der durch die tTG katalysierten Reaktion ein breites Spektrum an Acyl-Akzeptoren zur Verfügung steht, sind nur wenige Moleküle in der Lage als Acyl-Donoren zu fungieren. In einem in vitro Versuch konnte der durch die tTG vermittelte Einbau von radioaktiv markiertem Putrescin in Gliadin und damit die Funktion von Gliadin als Donor-Substrat der tTG nachgewiesen werden. In 160 µl Puffer (0,1 M Tris-HCl, 150 mM NaCl, 5 mM CaCl₂, pH 7,5) wurden 1 µg Substrat (Gliadin bzw. Kotrollproteine wie Albumin), 2 µCi [³H]-Putrescin und 1 µg tTG (aus Meerschweinchen, Sigma) 2 h bei 37 *°C* inkubiert. Die Reaktion wurde durch Zugabe von 100 µl 50 %-ige Trichloressigsäure (TCA) gestoppt und die Proteine wurden bei 4°C über Nacht präzipitiert. Nach Zentrifugation wurden die Pellets mit 10 %-iger TCA gewaschen, in SDS-Probenpuffer gelöst und einerseits in der SDS-PAGE aufgetrennt, andererseits zur Szitillationszählung verwendet. Während bei den Kontrollen kein Einbau an Putrescin festzustellen war, zeigte Gliadin sowohl durch die Daten der Szintillationszählung als auch in der SDS-PAGE einen deutlichen Einbau von [³H]-Putrescin in Gliadin, was belegt, daß Gliadin ein exzellentes Substrat für die tTG darstellt.

### Benutzte Abkürzungen

AK, Antikörper
APAAP, alkalische Phosphatase-Anti-Alkalische Phosphatase
BSA, Kinderserumalbumin
cm, Zentimeter
DMEM, Dulbecco's modifiziertes Eagle Medium
EC, Enzymkommission
ELISA, Enzuym-linked immunosorbent assay
EZM, Extrazelluläre Matrix
FKS, fötales Kälberserum
h, Stunde(n)
H₂O₂, Wasserstoffperoxid
HLA, humane Lymphozyten-Antigene
IEL, intraepitheliale Lymphozyten
Ig, Immunglobulin
kDa, Kilodalton
M, molar
mA, Milliampere
MHC, Haupthistokompatibilitätskomplex
mm, Minute(n)
mM, millimolar
Mᵣ, relative molekulare Masse
µg, Mikrogramm
µl, Mikroliter
PAGE, Polyacrylamidgeleledtrophorese
PBS, Phosphatpuffer
PLA₂, Phospholipase A₂
PVDF, Polyvinylidendifluorid
SDS, Natriumdodecylsulfat
TCA, Trichloessigsäure
TGF, transformierender Wachstumsfaktor
Tris, Tris-(hydroxymethyl)-aminomethan
tTG, Gewebe-Transglutaminase

## Patentansprüche

1. Verfahren zur Diagnose oder Therapiekontrolle der Sprue oder Zöliakie, **dadurch gekennzeichnet, daß** Antikörper gegen Gewebe-Transglutaminase (tTG) aus Körperflüssigkeiten durch eine Immunreaktion mit Gewebe-Transglutaminase (tTG), deren immunreaktiven Sequenzen oder Analoga nachgewiesen werden, wobei die Immunreaktion nicht mit einem Gewebeschnitt eines tierischen oder menschlichen Gewebes durchgeführt wird.

2. Verfahren nach Anspruch 1, **dadurch gekennzeichnet, daß** humane IgA-und/oder IgG-Antikörper nachgewiesen werden.

3. Verfahren nach Anspruch 1 oder 2, **dadurch gekennzeichnet, daß** die tTG humanen, tierischen, synthetischen oder rekombinanten Ursprungs ist.

4. Verfahren nach einem der Ansprüche 1 bis 3, **dadurch gekennzeichnet, daß** der Nachweis in einem an sich bekannten Immunoassay durchgeführt wird, vorzugsweise unter direkter oder indirekter Kopplung eines Reaktionspartners mit einer gut nachweisbaren Markierungssubstanz.

5. Verfahren nach Anspruch 4, **dadurch gekennzeichnet, daß** der Nachweis an einer festen Phase durchgeführt wind.

6. Verfahren nach Anspruch 4, **dadurch gekennzeichnet, daß** der Nachweis in einem ELISA, RIA oder Fluoreszenzimmunoassay erfolgt.

7. Verwendung von tTG, deren immunreaktiven Sequenzen oder Analoga zur Diagnose oder Therapiekontrolle der Sprue oder Zöliakie, wobei kein Gewebeschnitt eines tierischen oder menschlichen Gewebes verwendet wird.

8. Orales pharmazeutisches Mittel enthaltend tTG, deren immunreaktive Sequenzen oder Analoga und gegebenenfalls pharmazeutisch verträgliche Hilfsstoffe zur Behandlung der Sprue oder Zöliakie.

9. Verwendung von tTG, deren immunreaktiven Sequenzen oder Analoga zur Herstellung von oralen pharmazeutischen Mitteln zur Behandlung der Sprue oder Zöliakie.

## Claims

1. A method for the diagnosis or therapy control of sprue or coeliac disease, **characterized in that** antibodies against tissue transglutaminase (tTG) from body fluids are detected by means of an immune reaction with tissue transglutaminase (tTG), the immunoreactive sequences or analogues thereof, wherein the immune reaction is not carried out with a tissue section of animal or human tissue.

2. The method according to claim 1, **characterized in that** human IgA and/or IgG antibodies are detected.

3. The method according to claim 1 or 2, **characterized in that** the tTG is of human, animal, synthetic or recombinant origin.

4. The method according to any of claims 1 to 3, **characterized in that** the detection is performed using a *per se* known immunoassay, preferably with direct or indirect coupling of one reactant to a well-detectable marker substance.

5. The method according to claim 4, **characterized in that** the detection is carried out on a solid phase.

6. The method according to claim 4, **characterized in that** the detection is carried out using an ELISA, RIA or an immunofluorescence assay.

7. Use of tTG, the immunoreactive sequences or analogues thereof in the diagnosis or therapy control of sprue or coeliac disease, wherein no tissue section of animal or human tissue is employed.

8. An oral pharmaceutical agent including tTG, the immunoreactive sequences or analogues thereof and optionally pharmaceutically tolerable adjuvants for the treatment of sprue or coeliac disease.

9. Use of tTG, the immunoreactive sequences or analogues thereof in the production of oral pharmaceutical agents for the treatment of sprue or coeliac disease.

## Revendications

1. Procédé de diagnostic ou de contrôle de la thérapeutique de la sprue ou de la maladie coeliaque,
**caractérisé en ce qu'**
on détecte des anticorps contre la transglutaminase tissulaire (tTG) provenant des liquides corporels par une réaction immune avec de la transglutaminase tissulaire (tTG), ses séquences immuno-réactives ou ses analogues, procédé dans lequel la réaction immune n'est pas effectuée avec une coupe d'un tissu animal ou humain.

2. Procédé selon la revendication 1,
**caractérisé en ce qu'**
on détecte des anticorps contre IgA et/ou contre IgG humains.

3. Procédé selon l'une quelconque des revendications 1 ou 2,
**caractérisé en ce que**
la tTG est d'origine humaine, animale, synthétique ou recombinante.

4. Procédé selon l'une quelconque des revendications 1 à 3,
**caractérisé en ce que**
la détection est effectuée dans un immuno-essai connu en soi, de préférence par couplage direct ou indirect d'un partenaire de réaction ayant une substance de marquage facilement détectable.

5. Procédé selon la revendication 4,
**caractérisé en ce que**
la détection est effectuée sur une phase solide.

6. Procédé selon la revendication 4,
**caractérisé en ce que**
la détection s'effectue dans un test ELISA, un RIA, ou un immuno-essai en fluorescence.

7. Utilisation de tTG, de ses séquences immuno-réactives ou de ses analogues, en vue du diagnostic ou du contrôle de la thérapeutique de la sprue ou de la maladie coeliaque dans laquelle aucune coupe de tissu d'un tissu animal ou humain, n'est utilisée.

8. Agent pharmaceutique oral contenant de la tTG, ses séquences immuno-réactives ou ses analogues et le cas échéant des adjuvants pharmaceutiquement compatibles, pour le traitement de la sprue ou de la maladie coeliaque.

9. Utilisation de la tTG, de ses séquences immuno-réactives ou de ses analogues en vue de la préparation de compositions pharmaceutiques par voie orale, en vue du traitement de la sprue ou de la maladie coeliaque.
